# EUROPÄISCHE PATENTSCHRIFT

(11) **EP 1 107 751 B1**
(45) Veröffentlichungstag und Bekanntmachung des Hinweises auf die Patenterteilung: **04.06.2003**
(21) Anmeldenummer: 99953589.1
(22) Anmeldetag: 25.08.1999
(51) Int. Cl.: A61K 31/28, A61P 35/00

(54) **ARZNEIMITTEL ENTHALTEND PLATINKOMPLEXVERBINDUNGEN SOWIE DEREN VERWENDUNG**
MEDICAMENT CONTAINING PLATINUM COMPLEX COMPOUNDS AND THE USE THEREOF
MEDICAMENTS CONTENANT DE COMPOSES COMPLEXES DE PLATINE, ET LEUR UTILISATION

(30) Priorität: 25.08.1998 DE 19838547
(43) Veröffentlichungstag der Anmeldung: 20.06.2001
(73) Patentinhaber: Deutsches Krebsforschungszentrum Stiftung des öffentlichen Rechts, 69120 Heidelberg (DE); Ruprecht-Karls-Universität Heidelberg, 69120 Heidelberg (DE)
(72) Erfinder: AMTMANN, Eberhard, D-69121 Heidelberg (DE); SCHILLING, Gerhard, D-68526 Ladenburg (DE)
(74) Vertreter: Tanner, James Percival
(86) Internationale Anmeldenummer: DE9902656
(87) Internationale Veröffentlichungsnummer: WO00010543

(56) Entgegenhaltungen:
- CRACIUNESCU, D. G. ET AL: "Dual in vivo pharmacological activities (antitrypanosomal and antitumor ) of some new complexes of platinum(II) and platinum(IV)" AN. R. ACAD. FARM. (1992), 58(4), 529-49 , 1992, XP000910520
- OSA, TETSUO ET AL: "Synthesis and antitumor activity of cis-dichloroplatinum complexes coordinating nitrogen cyclic or sulfur compounds" CHEM. PHARM. BULL. (1986), 34(9), 3563-72 , XP000907123
- MARCHESELLI, L. ET AL: "Synthesis, characterization and evaluation of biological activity of palladium (II) and platinum (II) complexes with dithiocarbamic acids and their derivatives as ligands" EUR. J. MED. CHEM. (1993), 28(4), 347-52 , 1993, XP000907110
- SINDELLARI, L. ET AL: "Dithiocarbamates as antagonists of cisplatin toxicity" INORG. CHIM. ACTA (1987), 137(1-2), 109-13 ,1987, XP000910510
- MITCHELL, KATHRYN A. ET AL: "Isolation, reactivity, and molecular structure of bis(2,2'-bipyridine)(.mu.-N-acetyl-L-cyste inato-S)diplatinum: model of the interaction of platinum with protein sulfur residues" INORG. CHEM. (1993), 32(12), 2608-9 ,1993, XP000910511

## Beschreibung

Die Erfindung betrifft Platinkomplexe enthaltende Arzneimittel sowie deren Verwendung zur Therapie von Tumorerkrankungen.

Gegenwärtig werden Krebserkrankungen meist vor und/oder nach einer Operation mit einer medikamentösen Therapie oder Radiotherapie behandelt. Bei der medikamentösen Tumortherapie, der Chemotherapie, werden Verbindungen eingesetzt, bei denen das Krebswachstum auf verschiedene Weise beeinflußt wird. Bei der Chemotherapie kommt es aber oft zu schweren, für den Patienten unangenehmen Nebenwirkungen, wie z.B. Haarausfall, Übelkeit, Erbrechen, Müdigkeit, Schädigung des Knochenmarks und der weißen Blutkörperchen. Dies gilt insbesondere für die bisher eingesetzten Platinverbindungen, wie Cisplatin oder Carboplatin. Oft kommt es auch zu mehr oder weniger schweren Sekundärinfektionen. Ferner sprechen nicht alle Tumorarten auf eine Chemotherapie an, z.B. das Nierenzellkarzinom oder Tumore des Magen-Darm-Trakts.

Der Erfindung liegt somit die Aufgabe zugrunde, ein wirksames Medikament zur Behandlung von Krebserkrankungen bereitzustellen. Das Medikament soll in geringer Dosierung wirksam sein, für gesunde Zellen möglichst wenig toxisch sein und wenig Nebenwirkungen besitzen. Ferner soll sich das Medikament auch für die regionale Chemotherapie eignen und ambulant verabreicht werden können. Außerdem soll das Medikament auch das Rückfallrisiko senken. Femer soll das Medikament ohne Wirkungsverlust längere Zeit gelagert werden können.

Es wurde überraschenderweise gefunden, dass Komplexe aus Platin und Xanthogenat stabile Verbindungen mit einer ausgezeichneten antitumoralen Wirkung ergeben.

Folglich betrifft die vorliegende Erfindung gemäß einem Aspekt Arzneimittel, die Platinkomplexe der allgemeinen Formel (I) 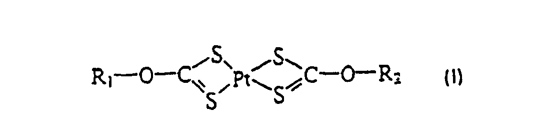 enthalten,
worin R₁ und R₂ jeweils unabhängig voneinander einen geraden oder verzweigten Alkylrest mit 1 bis 30 Kohlenstoffatomen, einen geraden oder verzweigten Alkenylrest mit 2 bis 30 Kohlenstoffatomen, einen mono- oder polyzyklischen Alkylrest mit 3 bis 30 Kohlenstoffatomen, einen mono- oder polyzyklischen Alkenylrest mit 4 bis 30 Kohlenstoffatomen, oder einen mono- oder polyzyklischen aromatischen Rest mit 6 bis 30 Kohlenstoffatomen bedeuten, wobei diese Reste gegebenenfalls durch einen oder mehrere Substituenten substituiert sein können.

Gemäß einem anderen Aspekt betrifft die vorliegende Erfindung die Verwendung des Arzneimittels bei der Herstellung eines Medikamentes zur Behandlung einer Krebserkrankung.

R₁ und R₂ können gleich oder verschieden sein.

Bevorzugt sind R₁ und R₂ gerade C₁₋₁₄-Alkylreste oder C₃₋₄-Cycloalkylreste. Besonders bevorzugt stehen R₁ und R₂ für CH₃CH_{2.}

Es kann jeder beliebige gerade oder verzweigte C₁₋₃₀-Alkylrest verwendet werden. Beispiele hierfür sind Methyl-, Ethyl-, Propyl-, Isopropyl-, Butyl-, Isobutyl-, tert.-Butyl-, n-Butyl-, n-Hexyl-, 2-Methylpentyl-, 2,3-Dimethylbutyl-, n-Heptyl-, 2-Methylhexyl-, 2,2-Dimethylpentyl-, 3,3-Dimethylpentyl-, 3-Ethylpentyl-, n-Octyl-, 2,2-Dimethylhexyl-, 3,3-Dimethylhexyl-, 3-Methyl-3-ethylpentylgruppen. Bevorzugt sind wegen der besseren Löslichkeit kurze Alkylketten, wie Methyl-, Ethyl-, Propyl- und Isopropyl-.

Es kann jeder beliebige gerade oder verzweigte C₂₋₃₀-Alkenylrest verwendet werden. Beispiele hierfür sind Vinyl-, Propenyl-, lsopropenyl-, Allyl-, 2-Methylallyl-, Butenyl- oder Isobutenyl-, Hexenyl- oder Isohexenyl-, heptenyl- oder Isoheptenyl-, Octenyl- oder lsooctenylgruppen. Bevorzugt sind Vinyl-, Propenyl- und Isopropenyl-.

Der Cycloalkylrest mit 3 bis 30 Kohlenstoffatomen kann jeder beliebige Cycloalkylrest sein. Beispiele hierfür sind eine Cyclopropyl-, Cyclobutyl-, Cyclopentyl- oder Cyclohexyl-, Cycloheptyl-, Cxclooctyl-, Cyclononyl- oder Cyclodecylgruppen. Bevorzugt sind Cyclopropyl-, Cyclobutyl-, Cyclopentyl- und Cyclohexyl-.

Der Cycloalkenylrest mit 4 bis 30 Kohlenstoffatomen kann jeder beliebige Cycloalkenylrest sein. Beispiele hierfür sind eine Cyclobutenyl-, Cyclopentenyloder Cyclohexenyl-, Cycloheptenyl-, Cxclooctenyl-, Cyclononenyl- oder Cyclodecenylgruppen. Bevorzugt sind Cyclobutenyl-, Cyclopentenyl- oder Cyclohexenyl. Beispiele für polyzyklische Alkyl- bzw. Alkenylreste umfassen Norbornan, Adamantan oder Benzvalen.

R₁ und R₂ können ferner beliebige mono- oder polycyclische C6-30- Arylreste sein. Beispiele hierfür sind ein carbocyclischer, monocyclischer Rest, beispielsweise die Phenylgruppe, ein heterocyclischer, monocyclischer Rest, beispielsweise die Gruppen Thienyl, Furyl, Pyranyl, Pyrrolyl, Imidazolyl, Pyrazolyl, Pyridyl, Pyrazinyl, Pyrimidinyl, Pyrazinyl, Thiazolyl, Oxazolyl, Furazannyl, Pyrrolinyl, Imidazolinyl, Pyrazolinyl, Thiazolinyl, Triazolyl, Tetrazolyl, sowie die Positionsisomeren des oder der Heteroatome, die diese Gruppen umfassen können, ein Rest bestehend aus carbocyclischen kondensierten Ringen, beispielsweise die Naphthylgruppe oder die Phenanthrenylgruppe, ein Rest bestehend aus kondensierten heterocyclischen Ringen, beispielsweise Benzofuranyl, Benzothienyl, Benzimidazolyl, Benzothiazolyl, Naphtho[2,3-b]thienyl, Thianthrenyl, Isobenzofuranyl, Chromenyl, Xanthenyl, Phenoxathiinyl, Indolizinyl, Isoindolyl, 3H-lndolyl, Indolyl, Indazolyl, Purinyl, Chinolizinyl, Isochinolyl, Chinolyl, Phthalzinyl, Naphthyridinyl, Chinoxalinyl, Chinazolinyl, Cinolinyl, Pteridinyl, Carbazolyl, ß-Carbolinyl, Acridinyl, Phenazinyl, Phenothiazinyl, Phenoxazinyl, Indolinyl, Isoindolinyl, Imidazopyridyl, Imidazopyridmidinyl oder auch die kondensierten polycyclischen Systeme bestehend aus heterocyclischen Monozyklen, wie beispielsweise vorstehend definiert, wie beispielsweise Furo[2,3-b]pyrrol oder Thieno[2,3-b]furan, und insbesondere die Phenyl-, Furylgruppen, wie 2-Furyl, Imidazolyl, wie 2-lmidazolyl, Pyridyl, wie 2-Pyridyl, 3-Pyridyl, 4-Pyridyl, Pyrimidinyl, wie Pyridmid-2-yl, Thiazolyl, wie Thiazol-2-yl, Thiazolinyl, wie Thiazolin-2-yl, Triazolyl, wie Triazolyl-2-yl, Tetrazolyl, wie Tetrazol-2-yl, Benzimidazolyl, wie Benzimidazol-2-yl, Benzothiazolyl, Benzothiazol-2-yl, Purinyl, wie Purin-7-yl, oder Chinolyl, wie 4Chinolyl.

Vorzugsweise vorhandene Substituenten der verschiedenen vorstehend angegebenen Reste können aus der folgenden Gruppe ausgewählt werden:
- Halogen: Fluor, Chlor, Brom, lod,
- Amino, Alkylamino, Dimethylamino oder Ethylamino, Dialkylamino, wie Dimethylamino, Diethylamino, Methylethylamino, wobei jeder dieser Dialkylaminoreste gegebenenfalls in Oxidform vorliegt,
- Aminoalkyl, wie Aminomethyl oder Aminoethyl,
- Dialkylaminoalkyl, wie Dimethylaminomethyl oder -ethyl,
- Dialkylaminoalkyloxy, wie Dimethylaminoethyloxy,
- Hydroxyl,
- freie, veresterte Carboxylgruppe, wie Alkoxycarbonyl, beispielsweise Methoxycarbonyl oder Ethoxycarbonyl, oder in ein Salz, beispielsweise durch ein Natrium- oder Kaliumatom überführt,
- Alkyl mit 1 bis 8 Kohlenstoffatomen, wie Methyl, Ethyl, Propyl, Isopropyl, Butyl, Isobutyl, tert.-Butyl, gegebenenfalls durch ein oder mehrere Halogenatom(e) substituiert, beispielsweise durch Fluor, wie Trifluormethyl,
- Oxo, Cyano, Nitro, Formyl,
- Acyl, wie Acetyl, Propionyl, Butyryl, Benzoyl,
- Acyloxy, wie Acetoxy oder ein Rest der Formel:

   -O-CO-(CH₂)ₙCO₂H,

   worin n = 1 bis 5,
- Alkoxy, wie Methoxy, Ethoxy, Propyloxy, Isopropyloxy, Butyloxy,
- Alkylthio, wie Methylthio, Ethylthio, Propylthio, Isopropylthio, Butylthio,
- Carbamoyl,
- Alkenyl, wie Vinyl, Propenyl,
- Alkinyl, wie Ethinyl, Propinyl und
- Aryl, wie Phenyl, Furyl, Thienyl.

Als Beispiele für derartige substituierte Reste können ein durch ein oder mehrere Halogenatom(e) substituierter Alkylrest, wie die Trifluormethyl-, Trifluorbutyl-, Pentafluorpropyl-, Pentafluorbutyl-, Pentafluorpentyl-, Heptafluorbutyl- oder Nonafluorbutylgruppe oder 2-Chlorethyl- genannt werden.

Verbindungen der obigen Formel (I) lassen sich insgesamt mit dem Begriff "Thioplatin-Verbindungen" beschreiben.

Vorzugsweise werden die Verbindungen der Formel (I) durch ein Verfahren hergestellt, das dadurch gekennzeichnet ist, daß man in an sich bekannter Weise eine Ligandenaustauschreaktion aus einem Platinkomplex, wie z.B. Cischlorodiamminplatin(ll), mit dem entsprechenden Xanthogenat vornimmt. Dies ist beispielsweise ein Verfahren, daβ dadurch gekennzeichnet ist, das man eine Verbindung der Formel 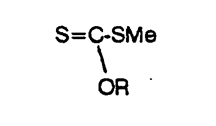 worin Me für ein Alkali- oder Erdalkalimetall steht, und R die für R₁ und R₂ angegebene Definition besitzt, mit einem Platinkomplex, wie cis-Dichiorodiamminplatin(ll), umsetzt und den erhaltenen neuen Platinkomplex isoliert.

Die Verbindungen der Formel (I) eignen sich zur Behandlung verschiedener Krebserkrankungen, wie z.B. Hodentumore, Ovarial-, Blasen-, Kolon-, Prostata-karzinome, klein- und nicht kleinzellige Bronchialkarzinome, Karzinome des Kopf- und des Halsbereichs, Karzinome des Thorax- und Abdomenbereichs, Zervixund Endometriumkarzinome, Sarkome und Melanome sowie Leukämien. Bevorzugt ist die Therapie des kleinzelligen Bronchialkarzinoms oder Kolorektalkarzinoms. Die Therapie kann auch als Begleittherapie zu einer Radiotherapie oder vor bzw. nach einem chirurgischen Eingriff erfolgen.

Die Verbindungen der Formel (I) sind gut verträglich. Der L₅₀-Wert ist um den Faktor 3 niedriger als für das in der Tumortherapie bekannte Cis-Platin. Bei einer Dosierung, die gute antitumorale Wirkungen hat, treten kaum Nebenwirkungen auf. Insbesondere ist bei den Thioplatin-Verbindungen die für Cis-Platin bekannte und gefürchtete Nephrotoxizität bisher in dieser Weise nicht aufgetreten. Ein weiterer Vorteil der erfindungsgemäßen Verbindungen ist, daß sie ein breites Wirkspektrum gegen verschiedenste Tumoren haben und insbesondere auch gegen Tumoren wirken, die bisher einer Therapie mit Platinverbindungen (z.B. Cisplatin) widerstanden. Thioplatin eignet sich insbesondere für solide Tumoren.

Ein weiterer Vorteil ist, daß die erfindungsgemäßen Verbindungen eine höhere Wirksamkeit im leicht sauren als im alkalischen pH-Wertbereich haben, da viele Tumorgewebe ein eher saures Milieu aufweisen. Von den Erfindern wurden Untersuchungen an Bis[0-ethyldithiocarbonato]platinum (II), einem Platin-Koordinationskomplex gemäß Formel (I) in dem Platin mit Schwefelatomen komplexiert ist, durchgeführt. Nach Protonierung öffnen sich zwei Schwefelligationen reversibel (so daß ein "Aqua-Komplex" gebildet wird), welche eine Vernetzung von DNA initiieren können. Nach Erhöhen des pH-Wertes dissoziieren die Protonen von den Schwefelatomem und das inerte Molekül wird wieder hergestellt. Es wird dafür folgende pH-abhängige Reaktionsgleichung aufgestellt:

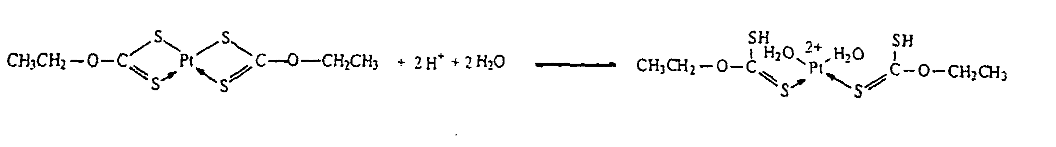

Aus dieser Gleichung ist ersichtlich, daß eine Verschiebung des pH-Wertes ins (leicht) Saure aus dem "Prodrug" gemäß Formel (I) die Entstehung der eigentlich reaktiven Verbindung bewirkt. Dieses Konzept wird durch die Beispiele bestätigt.

Das erfindungsgemäße Arzneimittel kann auf verschiedenen Wegen verabreicht werden, z.B. oral, parenteral, kutan, subkutan, intravenös, intramuskulär, rektal oder intratumoral. Bevorzugt ist die intravenöse oder intratumorale Verabreichung, d.h. die Verabreichung in bestimmte erkrankte Organe oder Körperregionen. Das Arzneimittel wird einem Patienten über einen vom Arzt zu bestimmenden Zeitraum verabreicht Das Arzneimittel kann sowohl Menschen als auch Säugern verabreicht werden.

Die Dosierung der erfindungsgemäßen Verbindung wird vom Arzt anhand der patientenspezifischen Parameter wie z.B. Alter, Gewicht, Geschlecht, Schwere der Erkrankung, etc. bestimmt. Bevorzugt beträgt die Dosierung zwischen 0,001 und 1000 mg/kg Körpergewicht.

Entsprechend der Art der Verabreichung wird das Arzneimittel in geeigneter Weise formuliert, z.B. in Form von einfachen oder dragierten Tabletten, Hart- oder Weichgelatinekapseln, Pulver zur Rekonstitution vor Gebrauch, Granulaten, Suppositorien, Ovula, Injektionspräparaten, Infusionslösungen, Pomaden, Cremes, Gels, Mikrosphären, Implantaten, die nach üblichen galenischen Verfahren hergestellt werden.

Die Verbindungen der Formel (I) können gegebenenfalls zusammen mit weiteren Wirkstoffen und mit in pharmazeutischen Zusammensetzungen üblichen Exzipientien formuliert werden, z.B. je nach herzustellendem Präparat Talk, Gummi arabicum, Lactose, Stärke, Magnesiumstearat, Kakaobutter, wäßrige und nichtwäßrige Träger, Fettkörper mit tierischem oder pflanzlichem Ursprung, Paraffinderivate, Glykole (insbesondere Polyethylenglykol), verschiedene Weichmacher, Dispergiermittel oder Emulgatoren, Konservierungsstoffe.

Zur Herstellung flüssiger Präparate können Additive wie Natriumchloridlösung, Ethanol, Sorbit, Glycerin, Olivenöl, Mandelöl, Propylenglycol oder Ethylenglycol verwendet werden.

Bevorzugt werden Infusions- oder Injektionslösungen hergestellt. Diese sind bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringe Mengen an Antibiotika können auch zugesetzt werden, um die Beibehaltung der Sterilität zu unterstützen.

Vorteilhaft ist die Bereitstellung des erfindungsgemäßen Arzneimittels in einer Dosis-Einheits-Form zur Verabreichung an einen Säuger, der eine Antikrebsbehandlung benötigt.

Die Erfindung betrifft auch Arzneimittel bzw. pharmazeutische Zusammensetzungen, die eine therapeutisch wirksame Menge des aktiven Inhaltsstoffs (erfindungsgemäße Verbindung der Formel (I)) zusammen mit organischen oder anorganischen inerten festen oder flüssigen pharmazeutisch verträglichen Trägem bzw. Verdünnungsmitteln, die für die beabsichtigte Verabreichung geeignet sind, und die mit den aktiven Inhaltsstoffen nicht nachteilig wechselwirken, enthalten.

Die Erfindung betrifft auch ein Verfahren zur Produktion einer pharmazeutischen Zusammensetzung, die dadurch gekennzeichnet ist, daß die Verbindung gemäß Formel (I) mit einem pharmazeutisch verträglichen Träger vermischt wird.

Unter den erfindungsgemäßen Medikamenten können insbesondere die im experimentellen Teil beschriebenen Verbindungen und ganz besonders die Verbindungen, bei denen in der obigen Formel (I) R1 und/oder R2, die gleich oder verschieden sein können, eine Methyl-, Ethyl-, Propyl- oder Isoproylgruppe ist, genannt werden.

Die erfindungsgemäßen Arzneimittel bzw. pharmazeutischen Zusammensetzungen umfassen als Wirkstoff mindestens einen wie vorstehend definierten Wirkstoff. Gegebenenfalls können noch weitere pharmazeutische Wirkstoffe in die Zusammensetzung aufgenommen werden, wie z.B. Immunsuppressiva, z.B. Cyclosporin, Rapamycin, 15-Desoxyspergualin, OKT3, Azathioprin; Zytokine (z.B. TNF), Interferon etc. Femer kann die erfindungsgemäße Zusammensetzung zusätzlich ein Steroid oder weitere Zytostatika (z.B. Cisplatin, Methotrexat, Aminopterin, Dacarbazin, Nitrosohamstoffverbindungen, Fluoruracil, Bleomycin, Daunomycin, Daunorubicin, Doxorubicin, Mithramycin, Mitomycin C, etc.) enthalten.

Die Erfindung wird weiter anhand der Figur erläutert:
- Fig. 1:: Tumorregression bzw. -wachstum nach Behandlung eines kleinzelligen Bronchialkarzinoms mit einer erfindungsgemäßen Verbindung bzw. Kontrolle
- Fig. 2:: Antitumorale Wirkung einer erfindungsgemäßen Verbindung auf ein menschliches Kolonkarzinom in der Nacktmaus
- Fig. 3:: pH-Wert abhängige Wirksamkeit von Zytostatika
- Fig. 4:: Anti-Tumor-Effekt von Thioplatin auf menschliche Xenotransplatate
- Fig. 5:: Histopathologie von Niere und Dünndarm nach Thioplatin- und Cis-Platin-Behandlung

Die Erfindung wird anhand der nachstehenden Beispiele näher erläutert.

### Beispiel 1

### Verfahren zur Herstellung von Diethylxanthogenat-platin(ll)-Komplexen (Bis-[O-ethyldithiocarbonato]platinum (II))

1 mmol cis-Dichlorodiamminplatin(ll) wurde in 600 ml destilliertem Wasser gelöst und unter Rühren mit 10 mmol Kaliumethylxanthogenat versetzt. Es wird bei Raumtemperatur sechs Stunden lang gerührt. Der entstandene Niederschlag wird abfiltriert, dreimal mit destilliertem Wasser gewaschen und im Vakuum getrocknet. Nach Umkristallisation aus warmem Aceton wird das Produkt als gelbe Kristalle mit einer Reinheit von mehr als 98% in einer Ausbeute von 68% erhalten.

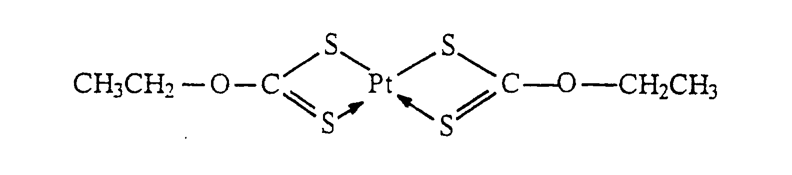

### Beispiel 2

Es wird die folgende pharmazeutische Zusammensetzung hergestellt:
Es wurden Injektionssuspensionen der folgenden Formulierung hergestellt:
   - Verbindung von Beispiel 1 g
   - Exzipiens (wäßrige Dispersionslösung): Benzylalkohol, Polyethylenglykol 900, Carboxymethylcellulose (Natriumsalz), Natriumchlorid, Wasser für ein Injektionspräparat für eine Ampulle zu 1 ml

Am bevorzugtesten ist PEG bis auf eine 5%ige Lösung zuzugeben, um die Lösung damit zu stabilisieren.

### Beispiel 3

### Pharmakologische Untersuchung mit der Verbindung gemäß Beispiel 1

### a) Antitumorale Wirkung auf ein menschliches kleinzelliges Bronchialkarzinom in der Nacktmaus

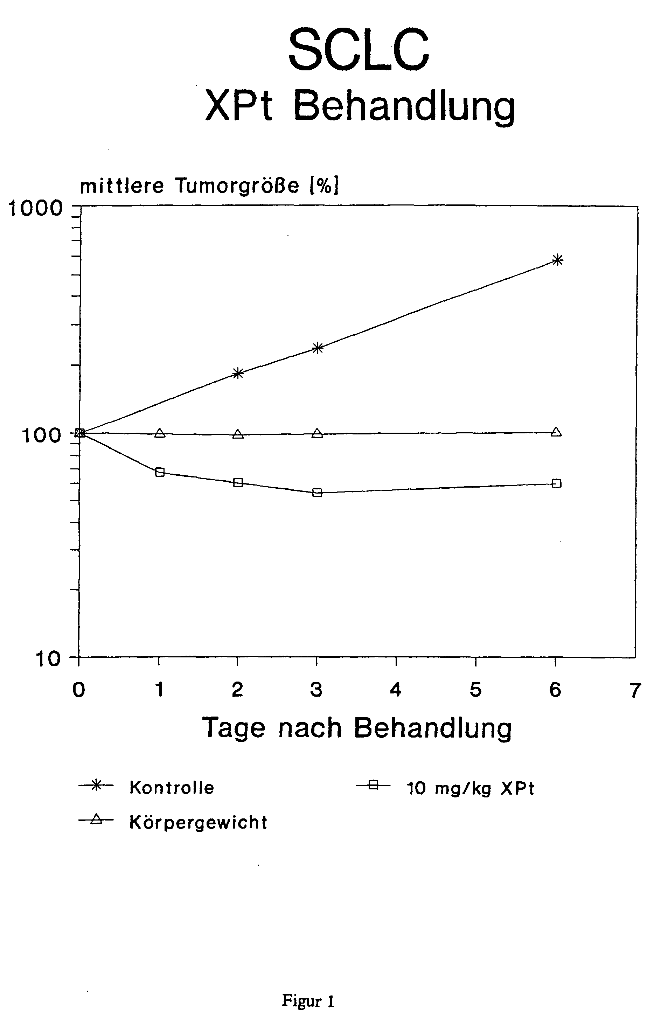

Bronchialkarzinomzellen (SCLC) wurden sechs Wochen alten Nu/Nu-Mäusen, Stamm NMRI (5x106 Zellen in 0,1 ml Kochsalzslösung) unter die Haut implantiert. Nach zwei Wochen hatten die Tumore eine Größe von etwa 8 mm erreicht. Dann wurden je fünf Tiere entweder mit 0,2 ml Kochsalzlösung/ 0,1%BSA/1% Aceton (Kontrollgruppe) oder mit 10 mg/kg Verbindung gemäß Beispiel 1 in Kochsalzlösung/1%BSA/1% Aceton (Behandlungsgruppe) i.v. behandelt. Das Tumorwachstum wurde täglich kontrolliert. Die Werte sind in der beigefügten Figur 1 dargestellt.

Man sieht deutlich, daß bei den mit der Verbindung gemäß Beispiel 1 behandelten Mäusen (Behandlungsgruppe) die Tumorgröße abnahm, während in den Mäusen der Kontrollgruppe das Tumorwachstum stetig stieg.

### b) Antitumorale Wirkung einer Verbindung gemäß Beispiel 1 auf ein menschliches Kolonkarzinom in der Nacktmaus

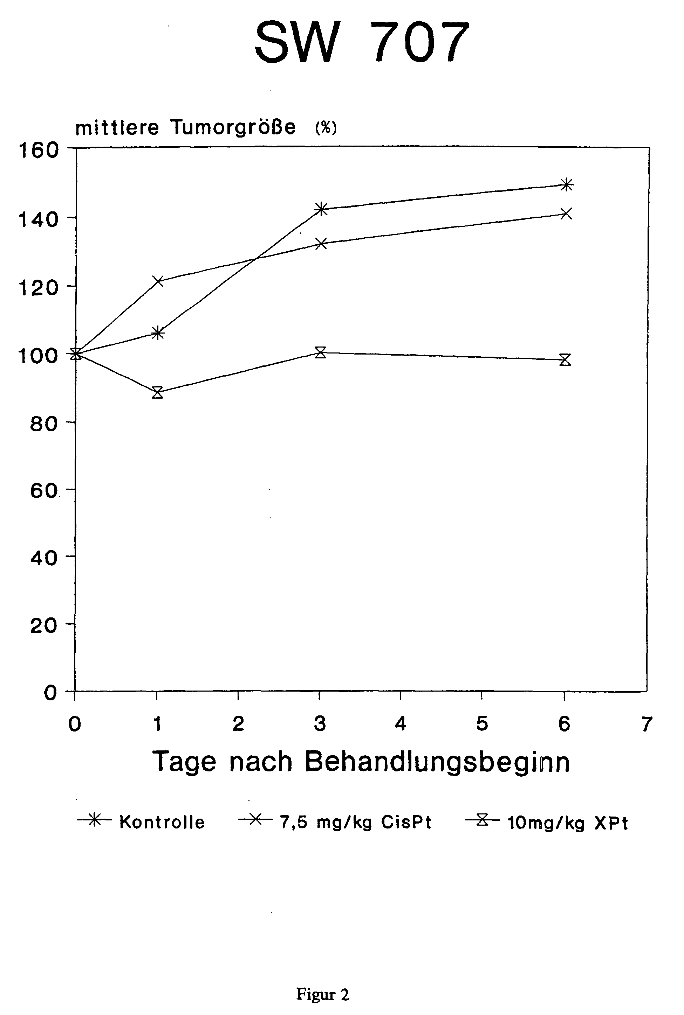

Kolonkarzinonzellen (SW707) wurden 6 Wochen alten Nu/Nu-Mäusen, Stamm NMRI (5 x 10⁶ Zellen in Kochsalzlösung) unter die Haut implantiert. Nach 10 Tagen als die Tumoren eine Größe von ca. 10 mm erreicht hatten, wurden je sechs Tiere entweder mit 0,2 ml Kochsalzlösung/1% BSA/1% Aceton (Kontrollgruppe), mit 7,5 mg/kg Verbindung gemäß Beispiel 1 in Kochsalzlösung/1% BSA/1% Aceton i.v. (Behandlungsgruppe 1) oder mit 10 mg/kg Cisplatin i.v. (Behandlungsgruppe II) behandelt. Das Tumorwachstum wurde täglich kontrolliert und in die in Fig. 2 gezeigte Graphik eingetragen.

Fig. 2 kann man entnehmen, daß das Tumorwachstum in der Behandlungsgruppe I gebremst wurde, während in der Kontrollgruppe und der Behandlungsgruppe ll ein stetiges Tumorwachstum zu beobachten war. Daraus kann man sehen, daß die erfindungsgemäßen Verbindungen Cisplatin weit überlegen sind und auch bei Tumoren eine Wirksamkeit entfalten, die bisher auf Platinverbindungen (Cisplatin) nicht ansprachen. Die Thioplatin-Verbindungen haben deshalb ein gegenüber bisherigen Zytostatika verbreiteres Wirkspektrum.

### Beispiel 4

### pH-Abhängigkeit der Wirksamkeit von Zytostatika

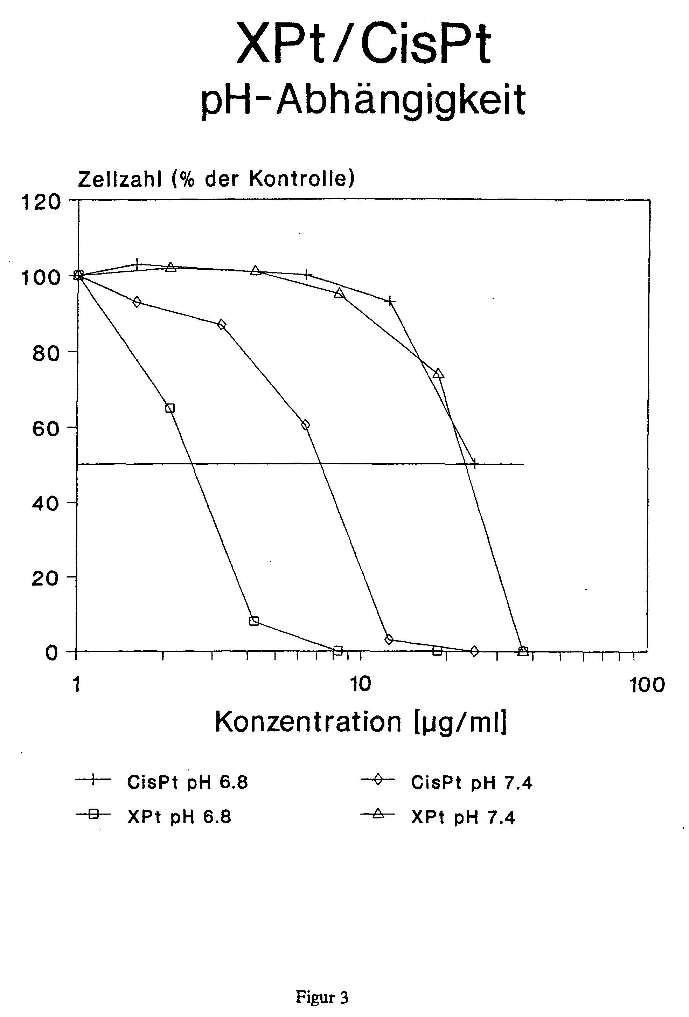

Hela-Zellen wurden in Linbro-Platten ausgesät (10⁵) und nach 6 Std. mit unterschiedlichen Konzentrationen von Cisplatin bzw. einer Verbindung gemäß Beispiel 1 in Basalmedium nach Eagle, das entweder 2,2 g (pH 7,4) oder 0,85 g (pH 6,8) Natriumbicarbonat und 10% fötales Kälberserum enthielt, für 24 Stunden bei 37°C in einer 5% CO₂-Atmosphäre inkubiert. Die Zahl der lebenden Zellen wurden nach Vitalfärbung mit Trypanblau in der Neubauer-Zählkammer bestimmt. Das Ergebnis ist in Fig. 3 zu sehen.

Überraschenderweise hat die Verbindung gemäß Beispiel 1 ein Wirkmaximum im leicht sauren Bereich, während das herkömmliche Zytostatikum Cisplatin im alkalischen Bereich besser wirkt. Da Tumorgewebe oft einen leicht sauren pH-Wert aufweist, ist klar, daß die Verbindung gemäß Beispiel 1 durch diese Eigenschaft eine höhere Wirksamkeit aufweist als herkömmliche Zytostatika.

### Beispiel 5

### pH-Abhängigkeit des Platin-Einbaus

10⁷ Hela-Zellen wurden in Petrischalen ausplattiert und 4 Stunden später wurden die Zellen mit einem Medium gefüttert, das entweder 2,2g/l (pH 7,4) oder 0,85 g/l (pH 6,8) NaHCO₃ enthielt. Nach Einstellung auf eine 5%ige C0₂-Atmosphäre wurden Cis-Platin oder Thioplatin auf jeweils eine Endkonzentration von 33 µM eingestellt. 4 Stunden nach dem Zusatz von Platin wurden jeweils eine Schale jeder Behandlungsgruppe 5 mal mit 50 ml kaltem PBS gewaschen. Die Zellen wurden von den Platten geschabt und der Pt-Gehalt wurde durch die Neutronen-Aktivierungs-Methode gestimmt. Dazu wurden nach dem Gefriertrocknen die Proben und Platin-Standards für 30 Minuten mit Neutronen (5 x 10¹² n cm⁻² sec⁻¹) bestrahlt. Eine Woche später wurde die γ-Strahlung von 158,4 keV und 208,2 keV bestimmt und die Platinkonzentration mit Hilfe von Standards bestimmt. Das Limit der Detektion war 2 ng.

An anderen Platten wurde 16 Stunden nach der Inkubation die DNA durch Phenol/Chloroform-Extraktion, gefolgt von einer Ethanol-Präzipitation, isoliert. Der Platin-Gehalt von jeweils 100 µg wurde wie vorbeschrieben mit der Neutronen-Aktivierungs-Methode bestimmt.

| Behandlung | Platin-Gehalt Gesamt-Zellen (ng/10⁷ Zellen) | Platin-Gehalt DNA (ng/mg) |
|---|---|---|
| unbehandelt, pH 6,8 | < 1 | <10 |
| unbehandelt, pH 7,4 | < 1 | <10 |
| CisPt, pH 6,8 | 140 | 14,6 |
| CisPt, pH 7,4 | 156 | 140 |
| ThioPt, pH 6,8 | 4780 | 13870 |
| ThioPt, pH 7,4 | 3680 | 1760 |

Aus den obigen Zahlen läßt sich entnehmen, daß nach der Inkubation mit Thioplatin bei einem leicht sauren pH-Wert von 6,8 eine fast 8-fach höhere Menge von Platin an DNA gebunden war als bei einer Inkubation bei pH 7,4. Mit Cis-Platin war der umgekehrte Effekt zu sehen, d.h. 10 mal mehr DNA wurde bei pH 7,4 an Cis-Platin gebunden als bei pH 6,8. Die Aufnahme von Platin durch die Zellen war pH-unabehängig. Jedoch war die Gesamtaufnahme 30-100 mal höher bei Verwendung von Thioplatin als bei Cisplatin.

### Beispiel 6

### Abhängigkeit der Cytotoxizität vom pH-Wert

Zellen wurden in Linbroschalen ausplattiert und 4 Stunden später wurden die Zellen mit einem Medium gefüttert, das entweder 2,2g/l (pH 7,4) oder 0,85 g/l (pH 6,8) NaHCO₃ enthielt. Nach Einstellung auf eine 5%ige C0₂-Atmosphäre wurden Cis-Platin oder Thioplatin auf jeweils Konzentrationen zwischen 5 und 150 µM zugegeben. Die Anzahl der lebenden Zellen wurde 24 Stunden später bestimmt und die lC₅₀₋ und lC₉₉-Werte aus den Dosis-Ansprechkurven berechnet (Standardabweichungen unter 10% in allen Fällen).

| Zellinie | Zelltyp | IC₅₀ pH 6.8/7.4 cisplatin (µM) | IC₉₉ pH 6.8/7.4 cisplatin (µM) | IC₅₀ pH 6.8/7.4 thioplatin (µM) | IC₉₉ pH 6.8/7.4 thioplatin (µM) |
|---|---|---|---|---|---|
| HeLa | menschl. Zervix-Krebs | 86/25 | 111/54 | 5/51 | 16.3/>55 |
| | | | | | |
| H10 | menschl. kleinzelliger Lungenkrebs | 72/42 | >150/90 | 5.1/15.4 | 13.2/30.8 |
| | | | | | |
| SW707 | menschl. Kolorectal-Krebs | 9.6/<4.8 | 90/38.4 | 3.7/13.2 | 7.5/26.4 |
| | | | | | |
| CV1 | Maus-Nierenkrebs | >150/>150 | >150/>150 | 3.9/23 | 13.2/106 |
| | | | | | |
| Capan2 | menschl. Pancreas- Krebs | 143/139 | >150/>150 | 17.3/50.6 | 40.9/99 |
| | | | | | |
| Dan-G | menschl. Pancreas-Krebs | 45/36 | 78/72 | 15.4/52.8 | 20.9/64.5 |
| | | | | | |
| Jurkat | menschl. T cell-Lymphoma | 52.5/54 | 120/126 | 6.8/21.1 | 13.6/132 |
| | | | | | |
| S180 | Maus-Sarcoma | 63/24 | >150/114 | 16.5/63.8 | 37.5/>110 |

Aus der Tabelle sieht man, daß die Cytotoxizität bei fast allen Zellinien für Thioplatin bei pH 6,8 viel besser war als bei pH 7,4, während bei Cisplatin die umgekehrten Ergebnisse erhalten wurden, d.h. bessere Wirksamkeit bei pH 7,4. In allen Fällen hatte Thioplatin eine 2-8 fach niedrigere lC₅₀ bei 6,8 als bei 7,4. Bei Cis-Platin war bei pH 6,8 die lC₅₀ schlechter als bei pH 7,4 oder es gab keinen nachweisbaren pH-Effekt.

### Beispiel 7

### Anti-Tumor-Effekt von Thioplatin auf menschliche Xenotransplatate

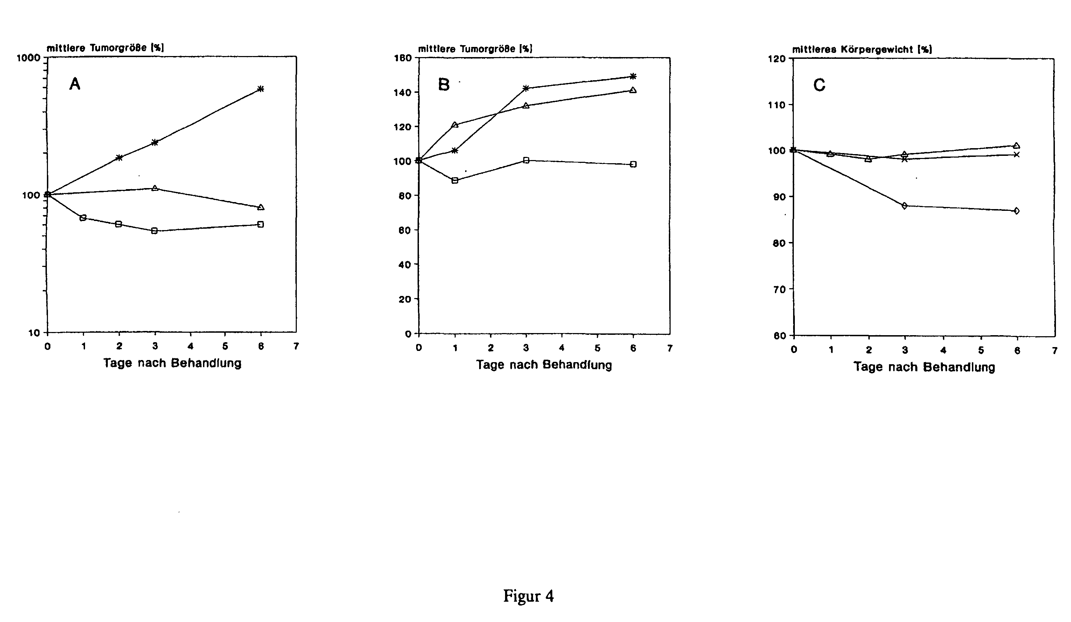

Es wird auf Fig. 4 verwiesen.

Menschliche H1O-Zellen eines kleinzelligen Lungenkarzinoms (A) oder SW707-Zellen eines Colorektal-Karzinoms (B) wurden subcutan in Nu/Nu-Swiss-Mäuse injiziert und 12 Tage später, wenn die Tumoren einen Durchmesser von 8-10 mm erreicht hatten, erhielten 5 Mäuse eine einzelne i.p Injektion von entweder 10 % Tween 80 (Sternchen; = Kontrolle), 10 mg/kg Cisplatin (Dreiecke) oder 10 mg/Kg Thioplatin gelöst auf eine Konzentration von 1 mg/ml in 10% Tween 80 (Quadrate). Die Tumorgrößen wurden in zwei Richtungen bestimmt und das relative Tumorwachstum wurden berechnet. Die Mittelwerte sind in Fig. 4 angegeben.

(C): Mittlere Körpergewichte der Gruppe mit kleinzelligen Lungenkarzinom. Die Standardabweichungen waren kleiner als 3%.

Aus Fig. 4 ist zu entnehmen, daß Cis-Platin bei H10-Zellen wirksam war, während beim Kolorektalkarzinom (SW707) sogar bei einer Dosis von 10 mg/kg keine Wirkung eintrat. In derselben Dosierung war Thioplatin bei beiden Krebsarten wirksam. Die mittlere Tumorgröße der Thioplatin-behandelten Gruppe war kleiner als die der Cis-Platin-behandelten Gruppe. Mit Thioplatin ging die Tumorgröße beim kleinzelligen Lungenkrebs auf 23% der Kontrollgruppe zurück (d.h. eine Verkleinerung auf ca. 1/4 der Kontrolle), während mit Cis-Platin nur eine Halbierung der Tumorgröße im Vergleich zur Kontrolle erreicht werden konnte (Fig. 4A). Im Fall von Kolorektal-Krebs zeigte Cis-Platin keinen Effekt, während das Tumorwachstum durch Thioplatin verhindert werden konnte (Fig. 4B).

Der Gewichtsverlust ist eine grobe Abschätzung der toxischen Nebenwirkungen. Während die Cis-Platin-behandelten Tiere einen Verlust von 12% und 13% an den Tagen 3 und 6 nach der Behandlung hatten, wurde in der Thioplatin-Gruppe kein Gewichtsverlust festgestellt (Fig. 4C).

### Beispiel 8

### Untersuchung der Toxizität auf Nieren und Dünndarm

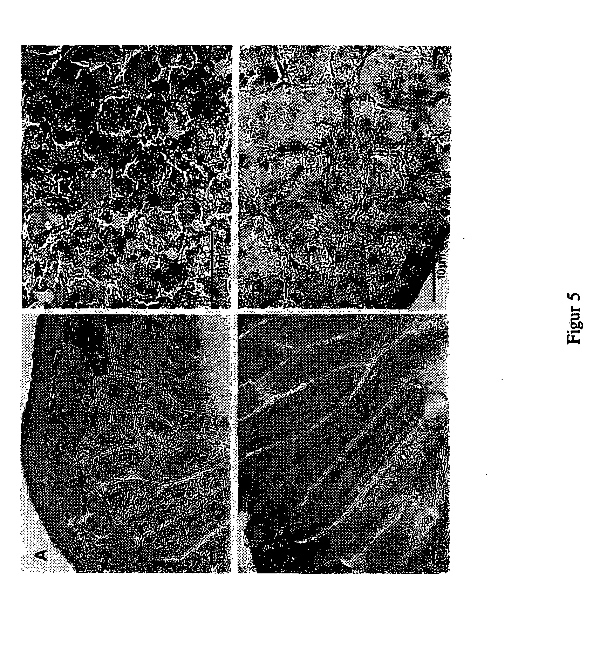

Es wird auf Fig. 5 verwiesen.

Swiss-Mäuse erhielten i.p. entweder 15 mg/kg Cisplatin (A,C) oder 20 mg/kg Thioplatin (B,D). Vier Tage später wurden histologische Schnitte von Dünndarm (A,B) und Nieren (C,D) angefertigt und mit Hämatoxylin und Eosin gefärbt.

In den Cis-Platin-behandelten Tieren zeigten die Nieren schwere Degeneration und Vakuolisierung der Tubuli (Fig. 5C). Die Struktur des Dünndarms ist größtenteils zerstört und es können in der Lamina propria (Fig. 5A) große Infiltrate gesehen werden. Im Gegensatz dazu, läßt die Behandlung mit einer hohen Dosis von 10 mg/kg Thioplatin die Struktur der Nieren (Fig. 5D) und des Dünndarms (Fig. 5B) unberührt.

## Patentansprüche

1. Arzneimittel **gekennzeichnet durch** einen Gehalt an mindestens einer Verbindung der allgemeinen Formel (I) 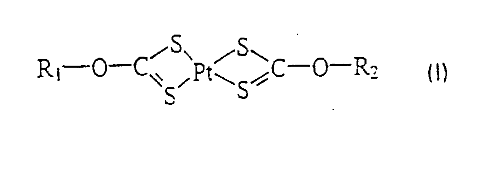 worin R₁ und R₂ jeweils unabhängig voneinander einen geraden oder verzweigten Alkylrest mit 1 bis 30 Kohlenstoffatomen, einen geraden oder verzweigten Alkenylrest mit 2 bis 30 Kohlenstoffatomen, einen mono- oder polyzyklischen Alkylrest mit 3 bis 30 Kohlenstoffatomen, einen mono- oder polyzyklischen Alkenylrest mit 4 bis 30 Kohlenstoffatomen, oder einen mono- oder polyzyklischen aromatischen Rest mit 6 bis 30 Kohlenstoffatomen bedeuten, wobei diese Reste gegebenenfalls **durch** einen oder mehrere Substituenten substituiert sein können.

2. Arzneimittel nach Anspruch 1, worin in der Verbindung der Formel (l) R₁ und R₂ jeweils für einen geraden C ₁₋₁₄-Alkylrest oder einen C₃₋₁₄-Cycloälkylrest stehen.

3. Arzneimittel nach Anspruch 1 oder 2, worin in der Verbindung der Formel (I) R₁ und R₂ jeweils für CH₃CH₂ stehen.

4. Arzneimittel nach einem der Ansprüche 1 bis 3, wobei die Verbindung der Formel (I) Dimethylxanthogenat-platin (II)-Komplex oder Diethylxanthogenat-platin (II)-Komplex ist.

5. Arzneimittel nach einem der Ansprüche 1-4, umfassend zusätzlich eine immunsuppressive Verbindung, ausgewählt aus Cyclosporin, Rapamycin, 15-Desoxyspergualin, OKT3 und Azathioprin.

6. Arzneimittel nach einem der Ansprüche 1-4, umfassend zusätzlich Zytokine, Interferon oder weitere Zytostatika.

7. Arzneimittel nach Anspruch 6, wobei das zusätzliche Zytostatikum Cisplatin, Methotrexat, Aminopterin, Dacarbazin, Nitrosoharnstoffverbindungen, Fluoruracil, Bleomycin, Daunomycin, Daunorubicin, Doxorubicin, Mithramycin, Mitomycin C ist.

8. Arzneimittel nach einem der Ansprüche 1-7, bereitgestellt in einer Dosis-Einheits-Form zur Verabreichung an einen Säuger, der eine Behandlung mit einem Antikrebsmit benötigt

9. Arzneimittel nach einem der Ansprüche 1-8, umfassend weiter einen pharmazeutisch verträglichen inerten Träger oder ein Verdünnungsmittel.

10. Verwendung eines Arzneimittels gemäß einem der Ansprüche 1-9 für die Herstellung eines Medikaments zur Behandlung einer Kerbserkrankung.

11. Verwendung nach Anspruch 10, wobei die Krebserkrankung ausgewählt ist aus Hodentumoren, Ovarial-, Blasen-, Kolon-, Prostata-karzinomen, klein- und nicht kleinzelligen Bronchialkarzinomen, Karzinomen des Kopf- und des Halsbereichs, Karzinomen des Thorax- und Abdomenbereichs, Zervix- und Endometriumkarzinomen, Sarkomen, Melanomen und Leukämien.

12. Verwendung nach Anspruch 10, wobei die Krebserkrankung das kleinzellige Bronchialkarzinom oder Kolorektal-Karzinom ist.

13. Verfahren zur Herstellung eines Arzneimittels nach einem der Ansprüche 1-9, **dadurch gekennzeichnet, daß** die Verbindung gemäß Formel (I) mit einem pharmazeutisch verträglichen Träger oder Verdünnungsmittel vermischt wird.

## Claims

1. A pharmaceutical preparation **characterized by** a content of at least one compound of general formula (I) 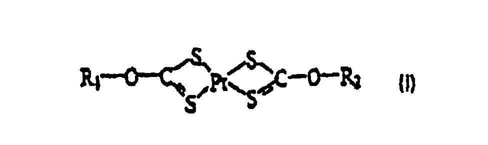 wherein R₁ and R₂ are each independently of each other a straight-chain or branched alkyl residue having 1 to 30 carbon atoms, a straight-chain or branched alkenyl residue having 2 to 30 carbon atoms, a monocyclic or polycyclic alkyl residue having 3 to /30 carbon atoms, a monocyclic or polycyclic alkenyl residue having 4 to 30 carbon atoms, or a monocyclic or polycyclic aromatic residue having 6 to 30 carbon atoms, these residues being optionally substituted by one or several substituents.

2. The pharmaceutical preparation according to claim 1, wherein in the compound of formula (I) R₁ and R₂ are respectively a straight-chain C₁₋₁₄ alkyl residue or a C₃₋₁₄ cycloalkyl residue.

3. The pharmaceutical preparation according to claim 1 or 2, wherein in the compound of formula (I) R₁ and R₂ are respectively CH₃CH₂.

4. The pharmaceutical preparation according to any one of claims 1 to 3, wherein the compound of formula (I) is dimethylxanthogenate platinum(II) complex or diethylxanthogenate platinum(II) complex.

5. The pharmaceutical preparation according to any one of claims 1 to 4, comprising additionally an immunosuppressive compound selected from the group consisting of cyclosporine, rapamycin, 15-deoxyspergualine, OKT3 and azathioprine.

6. The pharmaceutical preparation according to any one of claims 1 to 4, comprising additionally cytokines, interferon or further cytostatic agents.

7. The pharmaceutical preparation according to claim 6, wherein the additional cytostatic agent is cisplatin, methotrexate, aminopterin, dacarbacine, nitroso urea compounds, fluorouracil, bleomycin, daunomycin, daunorubicin, doxorubicin, mithramycin, mitomycin C.

8. The pharmaceutical preparation according to any one of claims 1 to 7, provided in a unit dosage form for administration to a mammal which requires treatment with an anticancer agent.

9. The pharmaceutical preparation according to any one of claims 1 to 8, further comprising a pharmaceutically compatible inert carrier or a diluent.

10. Use of a pharmaceutical preparation according to any one of claims 1 to 9 for the production of a medicament for treating a cancerous disease.

11. Use according to claim 10, wherein the cancerous disease is selected from testicular tumors, ovarian carcinomas, bladder carcinomas, colon carcinomas, prostatic carcinomas, parvocellular and non-parvocellular bronchial carcinomas, carcinomas of the cephalic and cervical parts, carcinomas of the thoracic and abdominal regions, cervical and endometrial carcinomas, sarcomas, melanomas and leukemias.

12. Use according to claim 10, wherein the cancerous disease is the parvocellular bronchial carcinoma or colorectal carcinoma.

13. A process for the production of a pharmaceutical preparation according to any one of claims 1 to 9, **characterized in that** the compound according to formula (I) is mixed with a pharmaceutically compatible carrier or diluent.

## Revendications

1. Médicament **caractérisé par** une teneur en au moins un composé de formule générale (I) 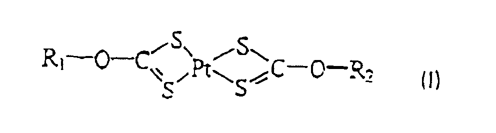 dans laquelle R₁ et R₂ représentent chacun, indépendamment, un reste alkyle linéaire ou ramifié ayant 1 à 30 atomes de carbone, un reste alcényle linéaire ou ramifié ayant 2 à 30 atomes de carbone, un reste alkyle monocyclique ou polycyclique ayant 3 à 30 atomes de carbone, un reste alcényle, monocyclique ou polycyclique ayant 4 à 30 atomes de carbone ou un reste aromatique monocyclique ou polycyclique ayant 6 à 30 atomes de carbone, ces restes pouvant éventuellement porter un ou plusieurs substituants.

2. Médicament suivant la revendication 1, dans lequel R₁ et R₂ représentent chacun un reste alkyle linéaire ayant 1 à 14 atomes de carbone ou un reste cycloalkyle ayant 3 à 14 atomes de carbone dans le composé de formule (I).

3. Médicament suivant la revendication 1 ou 2, dans lequel R₁ et R₂ représentent chacun un reste CH₃CH₂ dans le composé de formule (I).

4. Médicament suivant l'une des revendications 1 à 3, dans lequel le composé de formule (I) est un complexe de diméthylxanthogénate et de platine (II) ou un complexe de diéthylxanthogénate et de platine (II).

5. Médicament suivant l'une des revendications 1 à 4, comprenant en outre un composé immunosuppresseur choisi entre la cyclosporine, la rapamycine, la 15-désoxypergualine, le composé OKT3 et l'azathioprine.

6. Médicament suivant l'une des revendications 1 à 4, comprenant en outre des cytokines, un interféron ou d'autres cytostatiques.

7. Médicament suivant la revendication 6, dans lequel l'agent cytostatique, qui est en outre présent, est le cis-platine, le méthotrexate, l'aminoptérine, la dacarbazine, des composés nitrés de l'urée, le fluoruracile, la bléomycine, la daunomycine, la daunorubicine, la doxorubicine, la mithramycine, la mitomycine C.

8. Médicament suivant l'une des revendications 1 à 7, préparé sous une forme unitaire dosée destinée à être administrée à un mammifère qui nécessite un traitement avec un agent anti-cancéreux.

9. Médicament suivant l'une des revendications 1 à 8, comprenant en outre un support inerte acceptable du point de vue pharmaceutique ou un diluant.

10. Utilisation d'un médicament suivant l'une des revendications 1 à 9 pour la préparation d'un remède destiné au traitement d'une maladie cancéreuse.

11. Utilisation suivant la revendication 10, dans laquelle la maladie cancéreuse est choisie entre des tumeurs testiculaires, des cancers des ovaires, de la vessie, du colon, de la prostate, des cancers des bronches à cellules petites ou non, des cancers siégeant à la tête et au cou, des cancers siégeant au thorax et à l'abdomen, des cancers du col de l'utérus et de l'endomètre, des sarcomes, des mélanomes et des leucémies.

12. Utilisation suivant la revendication 10, dans laquelle la maladie cancéreuse est le cancer des bronches à petites cellules ou le cancer du colon et du rectum.

13. Procédé de préparation d'un médicament suivant l'une des revendications 1 à 9, **caractérisé en ce qu'**on mélange le composé de formule (I) avec un support ou un diluant acceptable du point de vue pharmaceutique.
